# EUROPEAN PATENT APPLICATION

(11) **EP 4 079 307 A1**
(43) Date of publication of application: **26.10.2022**
(21) Application number: 21169388.2
(22) Date of filing: 20.04.2021
(51) Int. Cl.: A61K 31/568, A61P 1/16

(54) **NEW MEDICAL USE**

(71) Applicant: Umecrine Cognition AB, 171 65 Solna (SE)
(72) Inventor: Doverskog, Magnus, 114 20 Stockholm (SE); Johansson, Maja, 911 92 Vännäs (SE); Öhman, Lars, 118 26 Stockholm (SE); Jones, David, Newcastle upon Tyne, NE20 0SU (GB)
(74) Representative: Hoffmann Eitle

(57) **Abstract**

The present invention is directed to the compound golexanolone or a pharmaceutically acceptable salt thereof, for use in the treatment of fatigue or cognitive impairment, or a combination thereof, in a non-cirrhotic patient with a chronic liver disease, wherein the patient has a serum level of allopregnanolone which is 0.03 ng/ml or higher.

## Description

### FIELD OF THE INVENTION

The present invention is directed to the compound golexanolone, having the chemical name 3α-ethynyl-3β-hydroxyandrostan-17-one oxime, or a pharmaceutically acceptable salt thereof, for use in the treatment of fatigue or cognitive impairment, or a combination thereof, in a non-cirrhotic patient suffering from a chronic liver disease, wherein the patient has a serum level of allopregnanolone which is 0.03 ng/ml or higher.

### BACKGROUND OF THE INVENTION

Chronic liver diseases that inevitably lead to hepatic fibrosis, cirrhosis and/or hepatocellular carcinoma have become a major cause of illness and death worldwide. Among them, cholangiopathies or cholestatic liver diseases comprise a large group of conditions in which injury is primarily focused on the biliary system. These include congenital diseases (such as biliary atresia and cystic fibrosis), acquired diseases (such as primary sclerosing cholangitis and primary biliary cholangitis), and those that arise from secondary damage to the biliary tree from obstruction, cholangitis or ischaemia. These conditions are associated with a specific pattern of chronic liver injury centered on damaged bile ducts that drive the development of peribiliary fibrosis and, ultimately, biliary cirrhosis and liver failure. For most, there is no established medical therapy and, hence, these diseases remain one of the most important indications for liver transplantation. Liver cirrhosis accounts for a growing and substantial disease burden worldwide *(*G Rajapaksha et al; World J Gastrointest Pathophysiol 2019; January 5; 10(1): pp. 1-10*).*

Biliary diseases or cholangiopathies are a group of chronic liver diseases characterized by cholestasis and progressive biliary fibrosis that can lead to end stage liver failure. There are numerous aetiologies for these diseases. Two of the common cholangiopathies are immune disorders, primary biliary cholangitis (PBC) and primary sclerosing cholangitis (PSC). Many cholangiopathies, including PBC, primarily affect the bile ducts. Once bile flow is impaired, bile is accumulated in the liver, causing primary damage to the biliary epithelium and eventually the liver parenchyma. A majority of cholangiopathies have similar features, including peri-portal inflammations that lead to liver fibrosis/cirrhosis. Given their progressive nature, most cholangiopathies cause substantial morbidity and mortality in patients and they are a major indication for liver transplantation.

Primary Biliary Cholangitis (PBC), previously known as primary biliary cirrhosis, is an autoimmune chronic disease of the liver. It results from a slow, progressive destruction of the small and intermediate bile ducts of the liver, causing bile and other toxins to build up in the liver, a condition called cholestasis, leading to i.a. periportal inflammation, fibrosis and biliary cirrhosis. PBC predominantly affects women. Characteristic symptoms include pruritus, fatigue and an increasingly recognised mild cognitive impairment. Cognitive symptoms are however prevalent in PBC independent of liver disease severity, and it is believed that PBC-related cognitive impairment is unrelated to hepatic encephalopathy *(*J.L. Newton et al; Hepatology, Vo. 48, No.2 2008pp. 541-549*).*

Primary sclerosing cholangitis (PSC) is a long-term progressive disease of the liver and gallbladder characterized by inflammation and scarring of the bile ducts which normally allow bile to drain from the gallbladder. The bile duct scarring which occurs in PSC narrows the ducts of the biliary tree and impedes the flow of bile to the intestines. Eventually, it can lead to cirrhosis of the liver and liver failure. PSC increases the risk of various cancers including liver cancer, gallbladder carcinoma, colorectal cancer, and cholangiocarcinoma. The underlying cause of PSC is unknown and there is no approved therapy available.

Fatigue is one of the most common non-specific symptoms considered as a crucial issue in health care and has been associated with several chronic disease states including multiple sclerosis, systemic lupus erythematosus, rheumatoid arthritis, as well as chronic viral and cholestatic liver diseases. A particularly high prevalence of fatigue in patients with primary biliary cholangitiss (PBC; 86%) and chronic hepatitis C (CHC; 69%) has been reported. The cause of fatigue associated with liver disease is not well understood, and few studies have investigated this symptom *(*S. Ahboucha et al: Neurogastroenterol Motil (2008); 20, pp. 671-679*).* Aboucha report significant increase of circulating levels of the neurosteroid allopregnanolone in cirrhotic patients with primary biliary cholangitis (PBC) and chronic hepatitis C (CHC).

In addition, sleep abnormality, in the form of excessive daytime somnolence, is present in a significant proportion of patients with PBC, with the degree of daytime somnolence correlating strongly with the degree of fatigue (Newton J.L. et al., Hepatology, Vo. 44, No.1 2006 pp. 91-98).

Fatigue is one of the most common symptoms of PBC. It occurs in up to 85% of patients (Huet PM, et al. Am J Gastroenterol. 2000; 95(3):pp. 760-767*),* and is a particularly debilitating symptom affecting quality of life and resulting in social isolation. No medical treatment has been shown to be effective in alleviating this symptom. Currently there are no widely accepted or effective treatments for fatigue in patients suffering from a chronic liver disease such as Primary Biliary Cholangitis (PBC), Primary sclerosing cholangitis (PSC) or any other type of cholangiopathy. Drugs such as Modafinil, Fluoxetine and Rituximab have been tried in patients suffering from PBC-related fatigue, but neither have proven beneficial.

The compound golexanolone, having the chemical name 3α-ethynyl-3β-hydroxyandrostan-17-one oxime, is a compound currently in clinical Phase II for the treatment of Hepatic Encephalopathy (HE). This compound is dislosed in WO 2008/063128 for use in various CNS disorders. WO 2015/114308 disclose the use of the compound 3α-ethynyl-3β-hydroxyandrostan-17-one oxime for the treatment of Hepathic Encephalopathy (HE). US patent application published as US2017/0348323, discloses a method for the treatment of hypersomnolence by administering the compound 3α-ethynyl-3β-hydroxyandrostan-17-one oxime.

R.F. Butterworth et al report that the effect of DHEAS (dehydroepiandrosterone sulphate) had a positive effect on whole body fatigue in rats following bile duct ligation (BDL) (R.F. Butterworth et al; Neurogastroenterol Motil (2009) 21, pp. 1319-1325*).* DHEAS is a steroid hormone which is active on several receptors such as GABA_{A}-receptors, NMDA-receptors, sigma-1 receptors and metabotrophic glutamate receptors (mGluR), and also modulates a variety of neurotransmitter systems, including the cholinergic, GABAergic, dopaminergic and glutamatergic systems (Y. Dong et al: Journal of Neuroendocrinology 2011; 24, pp. 215-224).

There is a medical need for a drug which may be useful in therapy of fatigue in a patient suffering from a chronic liver disease such as a cholangiopathy. There is also a medical need for a drug which may be useful in therapy of cognitive impairment in a patient suffering from a chronic liver disease such as a cholangiopathy.

### DESCRIPTION OF THE INVENTION

A problem underlying the present invention, is to find a novel therapy that may be useful for the treatment of fatigue or cognitive impairment, or a combination thereof, in a non-cirrhotic patient suffering from a chronic liver disease.

One aspect of the present invention, is the compound golexanolone (3α-ethynyl-3β-hydroxyandrostan-17-one oxime) of formula (I) 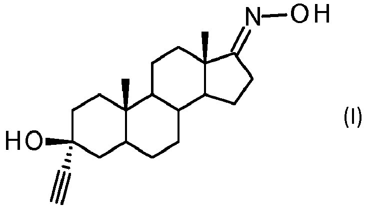 or a pharmaceutically acceptable salt thereof, for use in the treatment of fatigue or cognitive impairment, or a combination thereof, in a non-cirrhotic patient with a chronic liver disease, said patient having a serum level of allopregnanolone which is 0.03 ng/ml or higher.

In yet an aspect of the invention, the compound golexanolone (3α-ethynyl-3β-hydroxyandrostan-17-one oxime) is for use in the treatment of fatigue or cognitive impairment or excessive daytime sleepiness (EDS), or any combination thereof, in a non-cirrhotic patient with a chronic liver disease which is a cholangiopathy such as primary biliary cholangitis (PBC) or primary schlerosing cholangitis (PSC); chronic hepatitis B (CHB); chronic hepatitis C (CHC); non-alcoholic fatty liver disease (NAFLD); or non-alcoholic steatohepatitis (NASH);, and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with a cholangiopathy, and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with primary biliary cholangitis (PBC), and wherein the patient has a serum level of allopregnanolone which is 0.03 ng/ml or higher.

In one aspect of the invention, a non-cirrhotic patient with primary biliary cholangitis (PBC) as described and claimed herein, also suffers from itching.

In yet an aspect of the invention, a non-cirrhotic patient with primary biliary cholangitis (PBC) as described and claimed herein, also suffers from social problems.

In yet an aspect of the invention, a non-cirrhotic patient with primary biliary cholangitis (PBC) as described and claimed herein, also suffers from emotional problems.

In one aspect of the invention, the PBC patient as herein described and claimed has a PBC-40 Cognitive Domain which is 15 or higher(moderate).

In an aspect of the invention, the PBC patient as herein described and claimed has a PBC-40 Cognitive Domain which is in the range 15-21.

In yet an one aspect of the invention, the PBC patient as herein described and claimed has a PBC-40 Cognitive Domain which is 21 or higher (severe).

In one aspect of the invention, the PBC patient as herein described and claimed has a PBC-40 Fatigue Domain which is 28 or higher (moderate).

In yet an aspect of the invention, the PBC patient as herein described and claimed has a PBC-40 Fatigue domain which is in the range 28-39.

In yet an aspect of the invention, the PBC patient as herein described and claimed has a PBC-40 Fatigue domain which is 39 or higher (severe).

In yet an aspect of the invention, the PBC patient as herein described and claimed has a PBC-40 Itching Domain which is in the range 3-15.

In yet an aspect of the invention, the PBC patient as herein described and claimed has a PBC-40 Itching Domain which is 15 or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with primary schlerosing cholangitis (PSC), and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with chronic hepatitis C (CHC), and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with chronic hepatitis B (CHB), and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with nonalcoholic fatty liver disease (NAFLD), and wherein the patient has a serum level of allopregnanolone which is 0.03 ng/ml or higher.

Yet an aspect of the invention, is the compound golexanolone (3α-ethynyl-3β-hydroxyandrostan-17-one oxime) for use in the treatment of fatigue or cognitive impairment, or a combination thereof, in a non-cirrhotic patient with nonalcoholic steatohepatitis (NASH), and wherein the patient has a serum level of allopregnanolone which is 0.03 ng/ml or higher.

One aspect of the invention, is the compound golexanolone (3α-ethynyl-3β-hydroxyandrostan-17-one oxime) for use in the treatment of fatigue or cognitive impairment, or a combination thereof, in a non-cirrhotic patient with pre-existing cirrhosis, and wherein the patient has a serum level of allopregnanolone which is 0.03 ng/ml or higher.

One aspect of the invention, is a method for the treatment of fatigue or cognitive impairment, or a combination thereof, in a non-cirrhotic patient with a chronic liver disease, wherein the patient has a serum level of allopregnanolone which is 0.03 ng/ml or higher, whereby the compound golexanolone (3α-ethynyl-3β-hydroxyandrostan-17-one oxime) of formula (I) 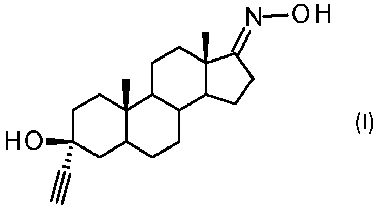 or a pharmaceutically acceptable salt thereof, is administered to a patient suffering from said chronic liver disease.

Yet an aspect of the invention, is the compound golexanolone (3α-ethynyl-3β-hydroxyandrostan-17-one oxime) for use in the treatment of a symptom related to cholangiopathy, such as primary biliary cholangitis (PBC) or primary sclerosing cholangitis (PSC).

Yet an aspect of the invention, is the compound golexanolone (3α-ethynyl-3β-hydroxyandrostan-17-one oxime) for use in the treatment of a symptom related to a chronic liver disease selected from chronic hepatitis B (CHB), chronic hepatitis C (CHC), nonalcoholic fatty liver disease (NAFLD), nonalcoholic steatohepatitis (NASH), and pre-existing cirrhosis.

Yet an aspect of the invention is a therapy against a symptom related to a chronic liver disease as herein disclosed and claimed, and which may provide a therapeutic effect.

Yet an aspect of the invention is the use of the compound golexanolone (3α-ethynyl-3β-hydroxyandrostan-17-one oxime) of formula (I) 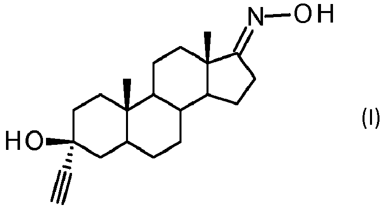 or a pharmaceutically acceptable salt thereof, for the manufacture of a medicament for use in the treatment of fatigue or cognitive impairment, or a combination thereof, in a non-cirrhotic patient with a chronic liver disease, wherein the patient has a serum level of allopregnanolone which is 0.03 ng/ml or higher.

In one aspect of the invention, the compound golexanolone (3α-ethynyl-3β-hydroxyandrostan-17-one oxime) is used as monotherapy.

In yet an aspect of the invention, the compound golexanolone (3α-ethynyl-3β-hydroxyandrostan-17-one oxime) may be used in combination with at least one more compound useful for the treatment of fatigue or cognitive impairment, or a combination thereof, in a non-cirrhotic patient suffering from a chronic liver disease and wherein the patient has a serum level of allopregnanolone which is 0.03 ng/ml or higher, such as a chronic liver disease selected from cholangiopathy such as primary biliary cholangitis (PBC) or primary sclerosing cholangitis (PSC), chronic hepatitis B (CHB), chronic hepatitis C (CHC), nonalcoholic fatty liver disease (NAFLD), nonalcoholic steatohepatitis (NASH), and pre-existing cirrhosis.

In one aspect of the invention, combination therapy may be as add-on therapy. In yet an aspect of the invention, the compound golexanolone (3α-ethynyl-3β-hydroxyandrostan-17-one oxime) may be administered as a fix combination (such as a pharmaceutical formulation) or by administration sequentially (i.e. after one another).

Medications which may be useful in combination therapy with the compound golexanolone (3α-ethynyl-3β-hydroxyandrostan-17-one oxime), is a medication or several medications, or a compound or several compounds, used as standard of care treatment of a patient with a chronic liver disease such as cholangiopathy, such as primary biliary cholangitis (PBC) or primary sclerosing cholangitis (PSC), chronic hepatitis B (CHB), chronic hepatitis C (CHC), nonalcoholic fatty liver disease (NAFLD), nonalcoholic steatohepatitis (NASH), pre-existing cirrhosis or cirrhosis.

Examples of medications or compounds which may be useful in combination therapy with the compound golexanolone (3α-ethynyl-3β-hydroxyandrostan-17-one oxime) of PBC-related fatigue or cognitive impairment, or a combination thereof, in a non-cirrhotic patient and wherein the patient has a serum level of allopregnanolone which is 0.03 ng/ml or higher, are oral antioxidants, fluvoxamine, fluoxetine, ondansetron, colchicine, UDCA (ursodeoxycholic acid), OCA (obeticholic acid), ciclosporin, nalmefene, modafinil, rituximab, lactulose, rifaximin, propranolol, furosemide, a fibrates (such as bezafibrate and fenofibrate), methotrexate, or any combination thereof. Budesonide is yet an example of a compound which may be used in combination therapy with the compound golexanolone as herein described and claimed.

The compound golexanolone (3α-ethynyl-3β-hydroxyandrostan-17-one oxime) may be prepared according to the method of preparation disclosed in WO 2008/063128.
Figure 1 illustrates the correlation between the severity of cognitive symptoms and the level of the neurosteroid allopregnanolone in serum of PBC patients.
Figure 2 illustrates the correlation between the severity of fatigue symptoms and the level of the neurosteroid allopregnanolone in serum of PBC patients.

### DEFINITIONS

Cholangipathy (or cholangiopathies) is defined as a group of diseases that mainly target the biliary tree and which includes a group of chronic liver diseases characterized by cholestasis and progressive biliary fibrosis that can lead to end stage liver failure. Cholangipathy (or cholangopathies) can be divided into several categories according to the pathogenetic mechanism involved. However, in many cholangiopathies, more than one pathogenetic mechanism is operative:
(1) Immune-mediated cholangiopathy
(2) Infectious cholangiopathy
(3) Genetic cholangiopathy
(4) Ischemic cholangiopathy
(5) Drug- or toxin-induced cholangiopathy

Two of the most common cholangiopathies are primary biliary cholangitis (PBC) and primary sclerosing cholangitis (PSC) respectively. PBC belongs to the group of immune mediated cholangiopathies.

Primary Biliary Cholangitis (PBC) is often considered a model of autoimmune disease because of its hallmark serologic signature, the antimitochondrial antibody and specific bile duct pathology. In addition to the risks of progression to cirrhosis and end-stage liver disease, PBC is characterised by significant symptoms of cognitive dysfunction and linked central fatigue which have a substantial impact on patient quality of life.

PBC patients experience a significant additional problem set with the development of central fatigue or cognitive dysfunction, or a combination of central fatigue and cognitive dysfunction, and related symptoms to these, in addition to the liver disease progression itself. The impact on quality of life is often significant, and is disproportionately high in younger patients adding to the level of disability. In contrast to disease progression, no progress has been made in treating this problem and this is a major area of unmet medical clinical need.

Cognitive symptoms, fatigue, excessive daytime sleepiness, or any combination thereof, , are present throughout cholangiopathy disease course in affected patients and are unrelated to conventional markers of disease severity, suggesting that they are unrelated to hepatic encephalopathy, and to advanced cirrhotic disease. There is also evidence of organic brain change in terms of impaired performance on formal cognitive testing, anatomical and functional change on MR, and neurophysiological assessment using transcranial magnetic stimulation.

Excessive Daytime Sleepiness (EDS) is defined as the inability to stay awake and alert during major waking episodes of the day, resulting in periods of irrepressible need for sleep or unintended lapses inta drowsiness or sleep. The Epworth Sleepiness Scale (ESS) is widely used in the field of sleep medicine as a subjective measure of a patient's sleepiness (Johns 1991; Sleep, 14(6), pp. 540-545). The total ESS score can range from zero to 24 with higher scores correlating with increasing degrees of sleepiness.

Primary sclerosing cholangitis (PSC) is a long-term progressive disease of the liver and gallbladder characterized by inflammation and scarring of the bile ducts which normally allow bile to drain from the gallbladder. The bile duct scarring which occurs in PSC narrows the ducts of the biliary tree and impedes the flow of bile to the intestines. Eventually, it can lead to cirrhosis of the liver and liver failure.

Chronic hepatitis is an inflammation of the liver that lasts for at least 6 months. Many people have no symptoms, but others have vague symptoms, such as a general feeling of illness, poor appetite, and fatigue. Chronic hepatitis can result in cirrhosis with portal hypertension and liver failure. Both chronic hepatitis B (CHB) and chronic hepatitis C (CHC) are caused by a virus infection characterised by varying degrees of inflammation and hepatic fibrosis.

Nonalcoholic fatty liver disease (NAFLD) is an umbrella term for a range of liver conditions affecting people who drink little to no alcohol. Excess fat builds up in the liver due to causes other than alcohol use. NAFLD is marked by liver inflammation, which may progress to scarring and irreversible damage. This damage is similar to the damage caused by heavy alcohol use.

Nonalcoholic steatohepatitis (NASH) is the most severe form of non-alcoholic fatty liver disease (NAFLD), and is characterized by the presence of an abnormal accumulation of fat in the liver which in some individuals can progress to liver cell injury (hepatocellular ballooning) and inflammation. As NASH evolves, over time it can result in excessive scarring in the liver (fibrosis), a natural response to injury which can lead to liver cirrhosis or liver cancer.

Cirrhosis is a progressive disease, developing slowly over many years. If it is allowed to continue, the buildup of scar tissue can eventually stop liver function. For cirrhosis to develop, long-term, continuous damage to the liver needs to occur. When healthy liver tissue is destroyed and replaced by scar tissue, the condition becomes serious, because it can start blocking the flow of blood through the liver.

Patients suffering from a chronic liver disease, often also suffers from symptoms such as fatigue, cognitive impairment or a combination of fatigue and cognitive impairment.

Fatigue in a patient suffering from a chronic liver disease including cholangiopathy such as primary biliary cholangitis (PBC) or primary sclerosing cholangitis (PSC), chronic hepatitis B (CHB), chronic hepatitis C (CHC), nonalcoholic fatty liver disease (NAFLD), nonalcoholic steatohepatitis (NASH), or pre-existing cirrhosis, are each within the scope of the present invention.

Patients suffering from a chronic liver disease such as PBC or PSC and who suffers from symptoms such as fatigue,or cognitive impairment (brain fog), or a combination thereof, has a pathophysiology which is different from a patient suffering from Hepathic Encephalopathy.

Characteristic symptoms of PBC include pruritus, fatigue and/or an increasingly recognised mild cognitive impairment. Cognitive symptoms are however prevalent in PBC independent of liver disease severity, and it is believed that PBC-related cognitive impairment is unrelated to hepatic encephalopathy (J.L. Newton et al; Hepatology, Vo. 48, No.2 2008; pp. 541-549).

Fatigue in patients suffering from PBC, have both peripheral and central fatigue components. The central component ("brain fog") is characterised by neurophysiological abnormalities and is related to cognitive symptoms and sleep pattern disturbance. The peripheral component means an inability to sustain physical activity, loss of energy or subjects feeling that they are "running out of fuel".

Cognitive impairment (reduced cognitive performance), whilst also being associated with fatigue, is a significant discrete symptom in patients suffering from PBC.

The disease terminology and symptom terminology as used throughout the specification and claims, is terminology in the field of chronic liver diseases applicable at the priority date of the present patent application. Terminology in the medical filed of liver diseases may evolve in the future for diseases such as PBC, NASH, schlerosing cholangitis (PSC), NAFLD, and NASH, but this should not change the scope of protection as herein described and claimed since the diseases and symptoms will still be the same.

The wording "treatment or therapy" as used throughout the specification and claims, takes the normal wording within the medical and pharmaceutical field, and means treatment of fatigue or cognitive impairment, or a combination thereof, in a non-cirrhotic patient who has been diagnosed as having (suffering from) a chronic liver disease as disclosed and claimed herein.

The wording "monotherapy" as used herein, means therapy with the compound golexanolone (3α-ethynyl-3β-hydroxyandrostan-17-one oxime) alone, administered to a non-cirrhotic patient who is diagnosed or has been diagnosed with a chronic liver disease as disclosed and claimed herein.

The wording "combination therapy" as used herein, means treatment (therapy) with the compound golexanolone (3α-ethynyl-3β-hydroxyandrostan-17-one oxime) in combination with an agent (drug) used as standard of care therapy.

Combination therapy with an agent (drug) used as standard of care therapy according to the invention, means that the compound golexanolone (3α-ethynyl-3β-hydroxyandrostan-17-one oxime) may be administered prior to, after (add-on) or simultaneously to the administration of an agent (drug) used as standard of care therapy for a chronic liver disease as herein disclosed and claimed.

Add-on therapy as used herein, is defined as combination therapy wherein the compound golexanolone (3α-ethynyl-3β-hydroxyandrostan-17-one oxime) is administered to a subject who is already being treated with a medication indicated for fatigue in a patient with a chronic liver disease such as cholangiopathy, such as primary biliary cholangitis (PBC) or primary sclerosing cholangitis (PSC), chronic hepatitis B (CHB), chronic hepatitis C (CHC), nonalcoholic fatty liver disease (NAFLD), nonalcoholic steatohepatitis (NASH), or pre-existing cirrhosis.

A fix combination as used herein, may in one aspect be defined as a combination where the compound golexanolone (3α-ethynyl-3β-hydroxyandrostan-17-one oxime) is formulated in admixture with at least one more compound useful in the treatment of fatigue in a patient suffering from a chronic liver disease selected from cholangiopathy such as primary biliary cholangitis (PBC) or primary schlerosing cholangitis (PSC), chronic hepatitis B (CHB), chronic hepatitis C (CHC), nonalcoholic fatty liver disease (NAFLD), nonalcoholic steatohepatitis (NASH), and pre-existing cirrhosis. In yet an aspect, a fix combination may be defined as a "kit of parts" combination where each active ingredient or formulation may be administered simultaneously (i.e. in parallell but as separate formulations) or one after the other (sequentially).

The wording "treatment of fatigue or cognitive impairment, or a combination thereof" as used throughout the present specification and claims, means that a patient suffering from a chronic liver disease as disclosed and claimed herein, may suffer from only the symptom fatigue or only the symptom cognitive impairment, or from both the symptoms fatigue and cognitive impairment.

As used throughout the present specification and claims, the compound golexanolone is the compound with the chemical name 3α-ethynyl-3β-hydroxyandrostan-17-one oxime, according to the chemical formula (I), 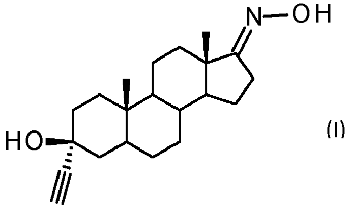 or a pharmaceutically acceptable salt thereof.

The wording "golexanolone" is the International Nonproprietary Name (INN) for the compound 3α-ethynyl-3β-hydroxyandrostan-17-one oxime, and this compound has the CAS no. 2089238-18-4.

The wording "serum" as used herein, means blood plasma without fibrinogens.

The wording "PBC-40" as used in accordance with the present invention, means a patient-derived, disease specific quality of life measure developed and validated for use in PBC (Jacoby A, Rannard A, Buck D, Bhala N, Newton JL, James OFW, Jones DEJ. Development, validation and evaluation of the PBC-40, a disease specific health related quality of life measure for primary biliary cirrhosis. Gut 2005;54: pp. 1622-1629). There are six domains of PBC-40 which relate to fatigue, emotional, social, cognitive function, general symptoms, and itch. The measure has been fully validated for use in PBC and shown to be scientifically sound. The wording "PBC-40 Fatigue Domain", "PBC-40 Cognitive Domain", PBC-40 Itching Domain", "PBC-40 Social Domain", and "PBC-40 Emotional Domain" respectively and as used in accordance with the present invention, are defined as disclosed in Jacoby et al (Gut 2005;54: pp. 1622-1629).

The wording "brain fog" as used herein, means difficulty in processing information, acting on it and remembering it.

### PHARMACEUTICAL FORMULATIONS AND ADMINISTRATION ROUTES

In certain aspects of the invention, the compound golexanolone (3α-ethynyl-3β-hydroxyandrostan-17-one oxime) as used throughout the present specification and claims, may be administered in the form of a pharmaceutical composition, in admixture with one or more pharmaceutically acceptable adjuvants, diluents and/or carriers. Examples of such pharmaceutically acceptable excipients, carriers and/or diluents useful when formulating the compound golexanolone (3α-ethynyl-3β-hydroxyandrostan-17-one oxime) for use in accordance with the present invention, are thickeners, flavoring agents, diluents, emulsifiers, dispersing aids, carrier substances, lubricants or binders. Typical pharmaceutical carriers include, but are not limited to, binding agents (e.g., pregelatinised maize starch); fillers (e.g., lactose, glucose, sucrose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, and sodium starch glycolate); wetting agents; diluents; coloring agents; emulsifying agents; pH buffering agents; preservatives; and mixtures thereof.

In one aspect of the invention, the compound golexanolone (3α-ethynyl-3β-hydroxyandrostan-17-one oxime) may be administered by enteral administration when used as disclosed and claimed herein. Examples of enteral administration involves administration to the esophagus, stomach, and small and large intestines (i.e., the gastrointestinal tract). Methods of administration include oral, sublingual (dissolving the drug under the tongue), and rectal.

The physician will be able to determine the actual dosage of the compound golexanolone (3α-ethynyl-3β-hydroxyandrostan-17-one oxime) which will be suitable for an individual patient in order to treat a chronic liver disease symptom selected from fatigue, cognitive impairment or a combination of both these symptoms, which dosage may vary with the route of administration, the type and severity of the symptom and patient population to be treated, as well as the species, age, weight, sex, and severity of the disease.

In one aspect of the invention, the compound golexanolone (3α-ethynyl-3β-hydroxyandrostan-17-one oxime) may be administered as a daily dose of from 1 mg to 200 mg, 10 mg to 100 mg, 3 mg to 30 mg, 30 mg to 60 mg, 50 mg to 100 mg, 20 mg to 160 mg, 40 mg to 160 mg, or 80 mg to 160 mg.
The wording "daily dose" may be administration of the compound golexanolone (3α-ethynyl-3β-hydroxyandrostan-17-one oxime) once daily, or twice daily (B.I.D.). Administration twice daily (B.I.D.) means that the total daily dose is divided into two doses which in total makes up the daily dose. For example, a daily dose of 1 mg to 200 mg may be administered as a dose of 1-200 mg once daily, or as a dose of 0.5-100 mg twice daily (B.I.D.).

In one aspect of the invention, a daily dose of the compound golexanolone (3α-ethynyl-3β-hydroxyandrostan-17-one oxime) which may be useful in accordance with the present invention may be selected from any one of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, and 160 mg.

### BIOLOGICAL EVALUATION

Inventors of the present invention have found and demonstrated that non-cirrhotic PBC patients with severe cognitive symptoms, have a significant elevation of the neurosteroid compound allopregnanolone in serum, and more particularly a serum level of allopreganolone which exceeds 0.03 ng/ml.

### Background and Aims

Rice S et al. report findings which indicate that fatigue, bone ache, and memory and concentration problems have the greatest impact on patient health-related quality of life (HRQoL) but being associated with low health service costs. In contrast, PBC complications have little additional effect on HRQoL while being associated with significant health service costs, except for ascites. These significant HRQoL and cost effects, accounting for current treatment, suggest the potential for benefit from new treatments (Rice S et al; Clin Gastroenterol Hepatol 2021; 19: pp. 768-776).

Allopregnanolone (AP), a neurosteroid, is a potent positive allosteric modulator of GABA_{A} receptors. It is linked to mood disorders with adverse effects on cognition and memory. This study explored associations between allopregnanolone (AP) and a disease-specific quality of life scoring system (PBC-40) in PBC patients.

### EXAMPLE 1

### Method

Serum AP levels were measured using mass spectrometry (detection limit 0.002ng/ml, quantitation limit 0.005ng/ml) in 160 subjects: 120 fully-phenotyped PBC patients from the UK-PBC (30) and BANC (90) studies and 40 age- and gender-matched healthy controls. All PBC patients completed the PBC-40 at recruitment. Serum AP was compared across the PBC-40 domains for those with no or mild symptoms (comparable to symptoms found in the general population) versus severe symptoms.

### Results

There were no significant differences in AP levels between healthy controls (median=0.03ng/ml [IQR=0.025]) and PBC (0.031 [0.42]), p=0.42, or between males (0.025 [0.033]) and females (0.032 [0.037]), p=0.57. Younger age was predictive of significantly higher AP levels within the PBC cohort (X2(2)=27.7, p<0.001) but not in healthy controls (X2(2)=6.0, p=0.119). There were significant differences within three PBC-40 domains: cognition (u=1034, p=0.02), emotional (u=1374, p=0.004) and itch (u=795, p=0.03). Younger age was associated with severe cognitive symptoms: severe (50 [12]) vs. none (60 [13]; p=0.001).

### Conclusion

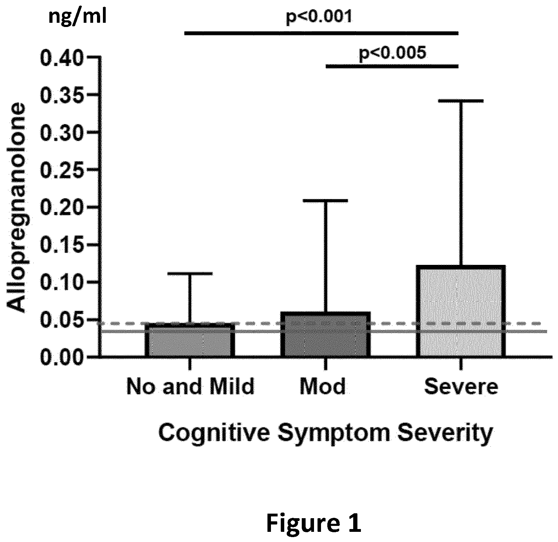

As shown in Figure 1, PBC patients having a higher serum level of allopregnanolone, have more severe cognitive symptoms. The solid horizontal line is the mean for the normals in the cohort, the dotted horizontal line the upper 95% Cl for the normal. The group "No and mild symptom" in Figure 1 are normal, which fits will all PBC-40 domains where "mild" symptoms are equivalent to ambient symptoms in the normal population. Severe are markedly affected and moderate are in the middle. Severe cognitive symptoms are only seen in a minority (10-15%) but are devastating, especially in younger patients. These are the people struggling to keep their jobs. The cut-offs for moderate and severe cognitive symptoms predict well the degree of Quality of Life (QoL) impairment (Jacoby et al; Gut 2005;54: pp. 1622-1629).

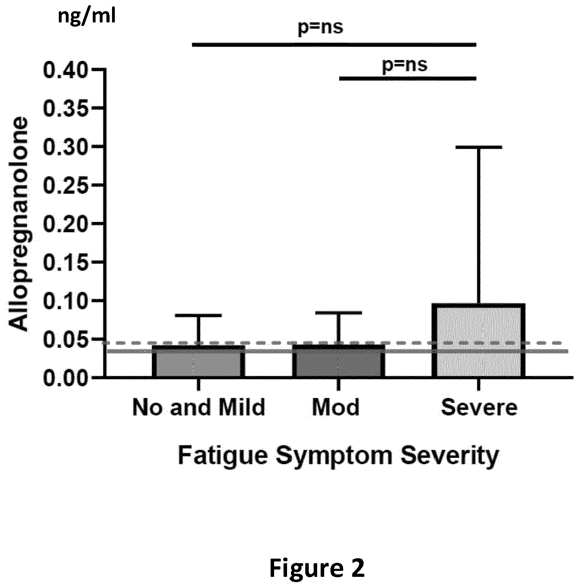

As shown in Figure 2, PBC patients having a higher serum level of allopregnanolone, have more severe Fatigue symptoms. The group "No and mild symptom" in Figure 2 are normal, which fits will all PBC-40 domains where "mild" symptoms are equivalent to ambient symptoms in the normal population. Severe are markedly affected and moderate are in the middle. Severe Fatigue symptoms are only seen in a minority (10-15%) but are devastating, especially in younger patients.

### EXAMPLE 2

### Assessing the effects of the compound golexanolone (3α-ethynyl-3β-hydroxyandrostan-17-one oxime) on fatigue and cognitive function in a Bile Duct Ligation (BDL) model of cholestatis

The BDL model is a validated model (Frissen et al. Hepatology 4 Aug 2020; https://doi.org/10.1002/hep.31494) which has previously been used to model fatigue in mice and has also been shown by Newcastle researchers to induce cognitive decline.

Cholestatic liver disease with moderate fibrosis is induced in mice using the bile duct ligation (BDL) model, where the bile duct is surgically ligated to restrict bile flow. Animals develop biliary obstruction that promotes inflammation and cholestasis followed by hepatocyte damage and then bile duct proliferation.

Assessment of the impact of the compound golexanolone (3α-ethynyl-3β-hydroxyandrostan-17-one oxime) on activity and cognitive function in the context of cholestatic liver disease is made.

### Animals

Adult C57BI6 male mice of 10 weeks of age with average weight of around 25 grams (Harlan UK), are used. The animals are maintained with 12:12h light-dark cycles with free access to food and water.

### BDL surgical procedure

Bile duct ligation (BDL) and sham surgery is performed under sterile conditions in a surgical procedure room. Animals are anaesthetized with isoflurane and buprenorphine is administered subcutaneously as an analgesic. In the sham surgical procedure, a laparotomy is performed followed by abdominal muscle and skin closure. The BDL procedure is as described for the sham surgery but laparotomy is followed by exposure and resection of the common bile duct, thereafter abdominal and skin wound closure. Immediately following surgery, all animals are housed in a heated laminar flow ventilated cabinet maintained at 25°C where they are carefully observed.

### Application and dosage of the compounds

Vehicle or the compound golexanolone (3α-ethynyl-3β-hydroxyandrostan-17-one oxime), is incorporated into diet for the duration of the study.

### Study groups

Three (3) different groups with n= 6/12 mice (total n=30) are included in the BDL model as shown below.

| **Group** | **Surgery** | **Treatment types** | **Treatment** | **Commence treatment** | **Follow up/harvest** | **Number of animals** |
|---|---|---|---|---|---|---|
| 1 | Sham | Vehicle | In diet | Day 0 | Day 10 | 6 |
| 2 | BDL | Vehicle | In diet | Day 0 | DaylO | 12 |
| 3 | BDL | golexanolone | In diet | Day 0 | Day 10 | 12 |

Mice are kept in a warm cabinet post surgery and monitored twice daily for physical condition. Bodyweight is measured at baseline and daily in order to ensure that no significant weight loss takes place due to surgery.

### Standardised behavioural testing

Mice are characterised using two standardised behavioural tests for the assessment of both short-term and long-term memory, as well as activity and exploratory behaviour on post-surgical days 4, 9 and 10.

The open field test (MouseTrapp, Neurolytical, USA) is performed at day 4 and 9. The open field test uses touchscreen technology to automatically record and score small animal movement and behaviour over a 5-minute duration.

Y-maze testing at day 10 is used to characterize cognitive dysfunction. The animal's preference for the 'novel' arm versus the familiar arms (proportional time in novel arm) is used as a measure of spatial memory.

Ethovision video tracking software is used to track movement of the mice in the open field test (anxiety/activity test; primary activity outputs - number of steps, distance travelled) and y-maze (short term/spatial memory test; percentage of time in novel arm).

### Organ Harvest

Brain tissue is collected (processed for histology and snap frozen) for future studies as may be required. Typical histological outputs may include - Immuno-fluorescence staining of neural populations e.g. neurons, interneurons, Sox2 positive progenitor, astrocytes or microglial cells.

Blood is taken for future studies as may be required. Typical outputs are AST (liver damage marker) and ALP (Biliary damage marker).

Bile is collected from the enlarged gall bladder and snap frozen for future studies as may be required (e.g. bile acid composition etc). Liver tissue is collected (processed for histology and snap frozen) for future studies as may be required. Typical histology outputs may include staining with Picrosirius Red and α-SMA to assess fibrosis and myofibroblast activation or keratin 19 (K19) for ductular proliferation.

Blood samples are drawn from the animals for analysis of the levels of allopregnanolone.

### EXAMPLE 3

### I. Clinical evaluation

The PBC-40 questionnaire is used in assessing fatigue and cognitive symptoms in PBC, using the disease specific PBC-40 scale with specific domains for cognitive function and fatigue respectively. The PBC-40 questionnaire may also include itching, social, emotional and general PBC related symptoms.

The PBC-40 questionnaire is a patient-derived, disease specific quality of life measure and questionnaire, which patients are asked to complete. The questionnaire consists of six so called domains of which cognitive function and fatigue are two such domains (the four other domains related to emotional, social, itch and general symptoms). The PBC-40 questionnaire is commonly used and understood by skilled persons within the medical field of PBC (Jacoby et al; Gut 2005;54: pp. 1622-1629).

### Study design

A phase II randomized, double-blind, dose-finding study of the compound golexanolone vs. placebo against background standard of care (SOC) among subjects with a history of non-cirrhotic or Childs A cirrhotic primary biliary cholangitis (PBC) with clinically significant fatigue and cognitive symptoms is performed. The study is conducted to evaluate the tolerability, safety and exploratory efficacy of golexanolone in this patient population.

### Investigational Medicinal Product(s), dosage and mode of administration

The Investigational Medical Product (clinical formulation) is supplied as a lipid semi-solid filled in a gelatine capsule for oral administration. For the present study, each capsule contains 10 mg golexanolone. The excipient is a mixture of mono -and diglycerides of capric/caprylic glycerides (Imwitor^{®} 742), which is of pharmacopoeia quality.

Placebo capsules contain the lipid excipient and is of identical appearance as the golexanolone capsules. The number of placebo capsules administeredare adjusted to match the number of golexanolone capsules administered on each dose level. The clinical formulation is described in the table below:

| | **Amount** | **Amount Capric/caprylic glycerides (Imwitor^{®} 742)** | **Clinical capsule dose** |
|---|---|---|---|
| **Compound I** | 10 mg | 490 mg | 10 mg capsule |
| **Placebo** | - | 500 mg | - |

The manufacture of the Investigational Medical Product formulation of golexanolone as used in the present clinical study, may be prepared as described in Example 10 of Applicant's published application WO2019102040.

The Investigational Medical Product golexanolone is administered daily for 28 consecutive days.

### Primary objective

The primary objective is to assess the safety and tolerability of golexanolone at day 28 in non-cirrhotic PBC patients with clinically significant fatigue compared to placebo.

### Secondary endpoint objectives

1. Assessment of the change in fatigue severity in study participants as assessed using the PBC-40 Fatigue Domain.
2. Assessment of the change in cognitive symptom severity as assessed by the PBC-40 Cognitive Domain.
3. Assessment of itching as assessed by the PBC-40 Itching Domain.
4. Assessment of change in Excessive Daytime Sleepiness (EDS) by the use of Epworth Sleepiness Scale (ESS).

Cognitive function change will be assessed by the use of informative tasks in the CANTAB platform.

### a) Planning: One Touch Stockings of Cambridge (OTS)

The participant sees two displays containing three colored balls and must work out in their head how many "moves" would be required to make the lower display match the upper display. Latency and accuracy measures are calculated.

### b) Visual episodic memory and learning: Paired Associates Learning (PAL)

Boxes are displayed on the screen and open in turn to reveal a number of patterns. The participant is asked to remember the location of each pattern. In the recall phase, each pattern is shown in the center of the screen, and the participant touches the box in which the pattern was located.

### c) Sustained Attention: Rapid Visual Information Processing (RVP)

Single digits appear one at a time at a rate of 100 digits per minute. Participants must detect a series of target sequences (e.g. 3-5-7) and touch a button when they see the last digit of a target sequence. Nine target sequences appear every 100 numbers.

### d) Working and memory strategy: Spatial Working Memory (SWM)

By process of elimination, the participant should find one "token" in each of the boxes on-screen and use them to fill up an empty column on the right hand side of the screen. The computer will never hide a token in the same coloured box, so once a token is found in a box the subject should not return to that box to look for another token.

### e) Emotion recognition: Emotion Recognition Task (ERT)

Images of faces showing a specific emotion are displayed on the screen, one at a time. Each face is displayed for 200 ms and then immediately covered up to prevent residual processing of the image. The participant must select which emotion face displayed from 6 options (sadness, happiness, fear, anger, disgust or surprise).

### f) Multitasking Test (MTT)

An arrow appears on either side of the screen (right or left) and can point in either direction (to the right or to the left). Each trial displays a cue at the top of the screen that indicates to the participant whether they have to select the right or left button according to the "side on which the arrow appeared" or the "direction in which the arrow was pointing".

### g) Processing speed and psychomotor speed: Reaction Time (RTI)

In five-choice reaction time test, the participant must press and hold down a touchscreen button at the bottom of the screen. A yellow spot will appear inside one of five yellow circles at the top of the screen. Participants must respond to the spot as quickly as they can by letting go of the button and touching the circle where the yellow spot appeared.

PBC patients normally have problems with: Paired learning (memory game): PAL; Working memory and strategy: SWM; Attention & data processing:RVP; Emotional recognition (what do faces say):ERT; Problem solving (first time):OTS; but usually less problems at Simple visual reaction time (RTI).

### Study design

Subjects are screened for eligibility according to study-specific inclusion/exclusion criteria prior to the first administration of the Investigational Medicinal Product (Visit 1; Screening visit). Eligible subjects will be randomized on Day 0 (Visit 2) to daily administration of:
10 mg golexanolone BID (i.e. 20 mg per day) in combination with Standard of Care therapy; or
40 mg golexanolone BID (i.e. 80 mg per day) in combination with Standard of Care therapy; or
80 mg golexanolone BID (i.e. 160 mg per day) in combination with Standard of Care therapy; or
placebo BID in combination with Standard of Care therapy.

Standard of Care therapy may be any one of an oral antioxidant, fluvoxamine, fluoxetine, ondansetron, colchicine, UDCA (ursodeoxycholic acid), OCA (obeticholic acid), ciclosporin, nalmefene, modafinil, rituximab, lactulose, rif Standard of Care aximin, propranolol, furosemide, methotrexate; or any combination thereof.

Each ranomized subject is included in one of the cohorts in the study. The treatment allocation (golexanolone or placebo) as well as the dose level is double-blinded, i.e. it is not disclosed to the subjects, site-staff nor Sponsor. The Investigational Medicinal Product is self-administered by the subject at home, except for the study days of visiting the clinic (Days 0, 14, and 28), when the study drug is administered under surveillance. Intake of study medication, changes in concomitant medications and medical events occurring between visits are registered in a diary on a daily basis by the patient.

Blood samples are drawn for serum levels of allopregnanolone.

A follow-up visit is performed on day 42 ± 5 days, i.e. 2 weeks after last administration of study drug.

### Inclusion Criteria

- Established diagnosis of PBC based on the presence of 2 out of the 3 key disease characteristics:
   ∘ AMA (anti-mitochondrial antibodies) or PBC-specific ANA (ant-inuclear antibodies) at a titre of 1/40 or greater;
   ∘ Elevated alkaline phosphatase (above the upper limit of normal (uln) for the relevant laboratory);
   ∘ Compatible or diagnostic liver biopsy;
- Clinically significant fatigue (defined for the purposes of this study as a PBC-40 Fatigue Domain score of ≥28 at screening) in the setting of clinically significant cognitive symptoms (PBC-40 Cognitive Domain ≥15);
- Stable UDCA dose for 3 months (either at 13-15mg/Kg) or not on UDCA if intolerant;
- For females of childbearing potential: Willing to use highly effective contraception or to practice sexual abstinence to avoid pregnancy for entire duration of the treatment period;
- Age ≥ 18 and ≤75

### Exclusion Criteria

- Liver cirrhosis Childs Pugh B or C disease;
- Clinical evidence of decompensation (hepatic encephalopathy or variceal bleeding);
- History of hepatocellular carcinoma;
- Bilirubin >1.5 x ULN;
- Evidence of biliary obstruction;
- Concomitant disease characterised by chronic fatigue ;
- Regular use of prescribed or over the counter medications known to cause fatigue or cognitive dysfunction (including, but not limited to, benzodiazepines, opiates other than codeine phosphate, sleeping pills, regular (daily) anti-histamine use (regular daily anti-histamine use in the last four weeks, anti-psychotic agents or recreational drug use);
- Anticipated change in PBC medication within the duration of the trial;
- Regular (more than one week per month) alcohol consumption in excess of recommended safe limits (14 units per week);
- Active participation in another interventional trial or exposure to another experimental drug within 5 half-lives;
- Pregnancy or planning to get pregnant;
- Clinical diagnosis of AIH overlap;
- Concurrent liver disease of another aetiology;
- Concurrent illness which in the opinion of the investigator is likely to compromise patient safety and/or interpretation of study results;

## Claims

1. The compound golexanolone (3α-ethynyl-3β-hydroxyandrostan-17-one oxime) of formula (I) 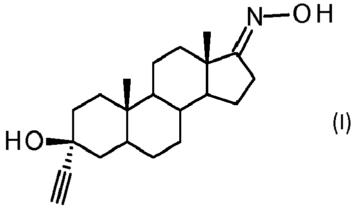 or a pharmaceutically acceptable salt thereof, for use in the treatment of fatigue or cognitive impairment, or a combination thereof, in a non-cirrhotic patient with a chronic liver disease, said patient having a serum level of allopregnanolone which is 0.03 ng/ml or higher.

2. The compound for use according to claim 1, wherein the chronic liver disease is a cholangiopathy.

3. The compound for use according to claim 2, wherein the cholangiopathy is primary biliary cholangitis (PBC).

4. The compound for use according to claim 3, wherein the PBC patient further suffers from itching.

5. The compound for use according to claim 3 or claim 4, wherein the PBC patient further suffers from social problems.

6. The compound for use according to any one of claims 3 to 5, wherein the PBC patient further suffers from emotional problems.

7. The compound for use according to any one of claims 1 to 6, wherein the patient further suffers from excessive daytime sleepiness (EDS).

8. The compound for use according to any one of claims 3 to 7, wherein the patient has a PBC-40 Cognitive Domaine which is
15 or higher (moderate), or
in the range 15-21, or
21 or higher (severe).

9. The compound for use according to any one of claims 3 to 8, wherein the patient has a PBC-40 Fatigue Domaine which is
28 or higher (moderate), or
in the range of 28-39, or
39 or higher (severe).

10. The compound for use according to claim 2, wherein the cholangiopathy is primary schlerosing cholangitis (PSC).

11. The compound for use according to claim 1, wherein the chronic liver disease is chronic hepatitis B (CHB) or chronic hepatitis C (CHC).

12. The compound for use according to claim 1, wherein the chronic liver disease is non-alcoholic fatty liver disease (NAFLD).

13. The compound for use according to claim 1, wherein the chronic liver disease is non-alcoholic steatohepatitis (NASH).

14. The compound for use according to any one of claims 1 to 13, for use in combination with at least one more compound useful in the treatment of a chronic liver disease.

15. The compound for use according to claim 14, wherein the at least one more compound is selected from anyone of an oral antioxidant, fluvoxamine, fluoxetine, ondansetron, colhicine, UDCA (ursodeoxycholic acid), OCA (obeticholic acid), ciclosporin, nalmefene, modafinil, rituximab, lactulose, rifaximin, propranolol, furosemide, methotrexate, or a fibrate (such as bezafibrate and fenofibrate); or any combination thereof.
